# EUROPÄISCHE PATENTANMELDUNG

(11) **EP 0 887 080 A2**
(43) Veröffentlichungstag der Anmeldung: **30.12.1998**
(21) Anmeldenummer: 98111615.5
(22) Anmeldetag: 24.06.1998
(51) Int. Cl.: A61K 35/20, A23L 1/305

(54) **Verwendung von Süssmolkekonzentrat zur selektiven Behandlung von Beta-hämolysierenden Streptococcen**

(30) Priorität: 24.06.1997 DE 19726873
(71) Anmelder: MK-Produktkontor GmbH, 58642 Iserlohn (DE)
(72) Erfinder: Krauskopf, Jobst, 58642 Iserlohn (DE)
(74) Vertreter: Hansen, Bernd, Dr. Dipl.-Chem.

(57) **Zusammenfassung**

Verwendung von Süßmolkekonzentrat zur Herstellung einer Zusammensetzung, die geeignet ist zur selektiven Bekämpfung β-hämolysierender Streptococcen und der durch diese verursachten Erkrankungen.

## Beschreibung

Die vorliegende Erfindung betrifft die Verwendung von Süßmolkekonzentrat zur Herstellung einer Zusammensetzung zur selektiven Bekämpfung β-hämolysierender Streptocoscen und der durch diese verursachten Erkrankungen.

Die gesundheitsfördernde Wirkung von Molke ist seit dem Altertum bekannt. Schon vor mehr als zweitausend Jahren wurde von Molkekuren berichtet, in Europa waren sie vor allem in der Zeit vom 17. bis 19. Jahrhundert beliebt, wobei die Förderung des allgemein Wohlbefindens durch Entgiftung und Entschlackung im Vordergrund stand. Auch im kosmetischen Bereich fand Molke Verwendung, wobei neben Molkekuren auch Molkebäder verabreicht wurden. In jüngster Zeit wurde die Molke, vor allem zur Anwendung in der modernen Diätetik, wiederentdeckt.

Molke fällt bei der Käseherstellung aus Milch, wie z.B. Kuhmilch, Ziegenmilch, Schafsmilch, Büffelmilch und Kamelmilch, an, nachdem das in der Milch vorhandene Casein ausgefällt wurde. Nach Art der Gewinnung unterscheidet man Süßmolke, die als Milchserum nach enzymatischer Ausfällung von Casein durch Labferment entsteht, und Sauermolke, die nach Abtrennung des Gaseins durch Säurefällung gewonnen wird. Die pH-Wert-Grenze zwischen Süß- und Sauermolke ist nicht ganz scharf umrissen und liegt allgemein über bzw. unter einem Bereich von 5,6 bis 5,9.

Molke enthält alle wasserlöslichen Komponenten der Milch, insofern diese nicht durch Lab oder Säure ausgefällt werden.

Süßmolke enthält ca. 4,9% Lactose, 0,8% Protein, 0,5% Mineralien, 0,2% Milchfett und 0,2% Milchsäure. Wichtige Proteine der Molke sind α-Lactalbumin, β-Lactoglobulin, Lactoferrin, Blutserumalbumin, Immunglobuline und Lactoperoxidase. An Fetten liegen sowohl gesättigte als auch ein- und mehrfach ungesättigte Fettsäuren sowie Cholesterin vor.

Weitere wichtige Inhaltsstoffe der Molke sind Vitamine (z.B. Ascorbinsäure, Thiamin, Niacin, Pantothensäure, Retinol, Riboflavin, Pyridoxin und Cobalamin) und freie Aminosäuren.

Süßmolke kann weiterhin teilweise oder vollständig hydrolysiert vorliegen, d.h. die Zucker sind dann in die entsprechenden Einfachzucker gespalten. Derartige Hydrolysate sind von der erfindungsgemäßen Bedeutung der Begriffe Süßmolke und Süßmolkekonzentrat mit umfaßt.

Bei allen Mengenangaben kann der Gehalt der Molke je nach verwendeter Milch (z.B. Kuh, Ziege, Schaf) und nach hergestellter Käseart schwanken.

Aufgrund ihres Nährstoffgehalts eignet sich Molke zur Herstellung ernährungsphysiologisch wertvoller Produkte. Der hohe Lactosegehalt der Molke wirkt sich günstig auf die endogene Verwertung von Calcium, Natrium und Kalium aus der Nahrung aus, und die durch Molke erhöhte Vitamin- und Mineralstoffzufuhr ist insbesondere bei älteren mangelernährten Menschen wünschenswert.

Aus der medizinischen Literatur ist bekannt, daß sich in der Mundhöhle des Menschen viele Bakterienarten halten können. Die Bakterien gelangen mit der Nahrung in die Mundhöhle und finden dort Bedingungen vor, unter denen sie sich dort halten und weiter vermehren können.

Eine Gruppe von Bakterien, die sich dort ansiedeln können, sind die Streptococcen. Die Streptococcen gehören zur Ordnung der Eubacteriales, Familie Lactobacillaceae, Gattung Streptococcaceae. Im mikroskopischen Bild zeigen sich Kugeln (Coccen) mit einem Durchmesser von etwa 0,5 bis 1 µm, die paarweise oder in Ketten angeordnet sind. Es handelt sich um mikroaerophile bis aerobe Mikroorganismen, die bei 37°C optimal wachsen und grampositiv sind.

In der Natur kommen Streptococcen auf Pflanzen vor sowie in Darm und Schleimhäuten von Mensch und Tier. Streptococcus lactis wird in der milchverarbeitenden Industrie als Säure-und Geschmackstoffbildner eingesetzt. Streptococcus salivarius bildet aus Saccharose Polyfructosen (Laevane).

Neben den nützlichen Vertretern dieser Gattung sind aber auch Schädlinge bekannt. Hier ist besonders der Vertreter Streptococcus mutans zu nennen, der als Erreger von Karies angesehen wird. Als Folge des Stoffwechsels dieses Mikroorganismus entsteht in der Mundhöhle ein saures Milieu. Dadurch wird der Zahnschmelz angegriffen, es entsteht Karies.

Streptococcen vom Typ mutans gehören zu den β-hämolysierenden Streptococcen, und besitzen die Eigenschaft, daß sie durch ihren Stoffwechsel die Bildung eines sauren Milieus fördern.

Die Aufgabe der vorliegenden Erfindung liegt darin, eine Zusammensetzung aufzufinden, die zur selektiven Bekämpfung β-hämolysierender Streptococcen und der durch diese hervorgerufene Erkrankungen in der Lage ist.

Es wurde überraschend gefunden, daß durch die Verwendung von Süßmolkekonzentrat (SMK) gemäß Anspruch 1 eine Zusammensetzung erhalten wird, die bei oraler Verabreichung zu einer selektiven Absenkung der in der Mundflora enthaltenen Zahl an β-hämolysierenden Streptococcen führt, wohingegen der Einfluß auf die Gesamtkeimzahl gering ist.

In einer weiteren Ausführungsform wird das SMK zur Herstellung einer Zusammensetzung verwendet, die weitere, zur Herstellung von diätetischen und/oder angereicherten Lebensmitteln geeignete Zusatzstoffe und/oder pharmazeutisch annehmbare Zusatzstoffe und/oder Trägermaterialien umfaßt.

Erfindungsgemäß kann das SMK verwendet werden zur Herstellung von Lebensmitteln, wie z.B. diätetischen Lebensmitteln und Nahrungsergänzungsmitteln, wodurch diese die Eigenschaft erhalten, daß sie bei oraler Verabreichung eine selektive Bekämpfung β-hämolysierender Streptococcen bewirken können. Beispiele für derartige Lebensmittel sind mit SMK angereicherte Milchprodukte oder Fruchtsäfte.

Ferner kann das SMK erfindungsgemäß verwendet werden zur Herstellung von pharmazeutischen Zusammensetzungen zur Behandlung von Erkrankungen, die durch β-hämolysierende Streptococcen hervorgerufen und/oder unterstützt werden.

Ferner ist das SMK erfindungsgemäß verwendbar zur Behandlung von durch β-hämolysierende Streptococcen hervorgerufene Erkrankungen, bevorzugt zur Behandlung solcher Erkrankungen bei Säugern, insbesondere beim Menschen. Bevorzugt ist die Verwendung zur Behandlung von Karieserkrankungen. Die Dosierung der durch die erfindungsgemäße Verwendung von

SMK erhaltenen Zusammensetzung kann je nach Alter, Gewicht und Geschlecht der zu behandelnden Person variieren. Bei der Verabreichung der Zusammensetzung an Säuger im allgemeinen erfolgt die Dosierung ebenfalls in Abhängigkeit von Tierart und Gewicht.

### Kurze Erläuterung der Figuren:

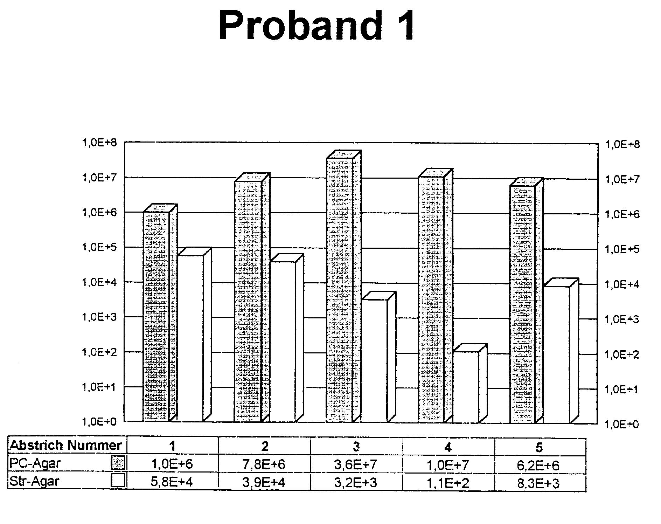

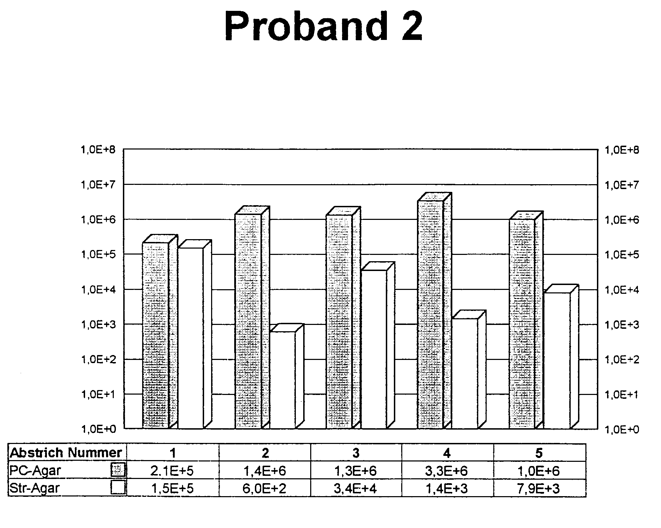

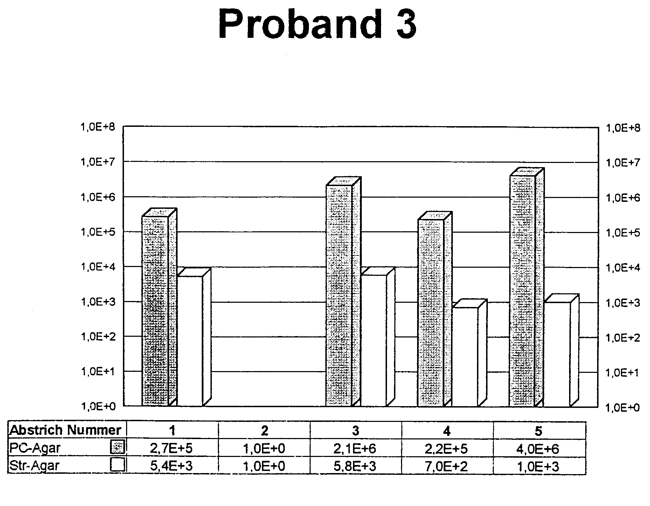

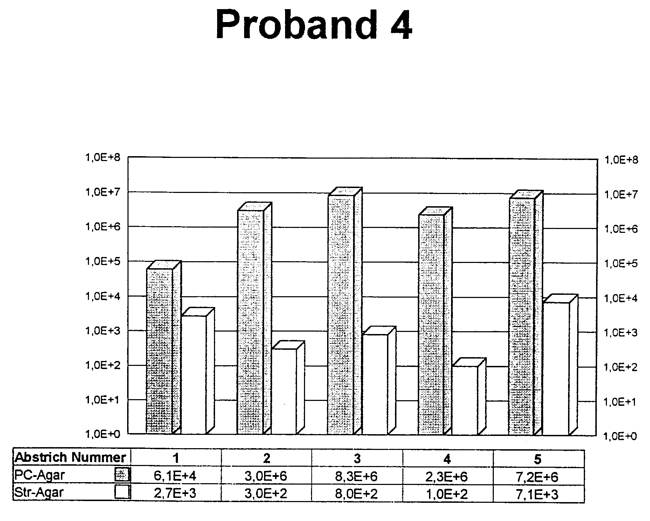

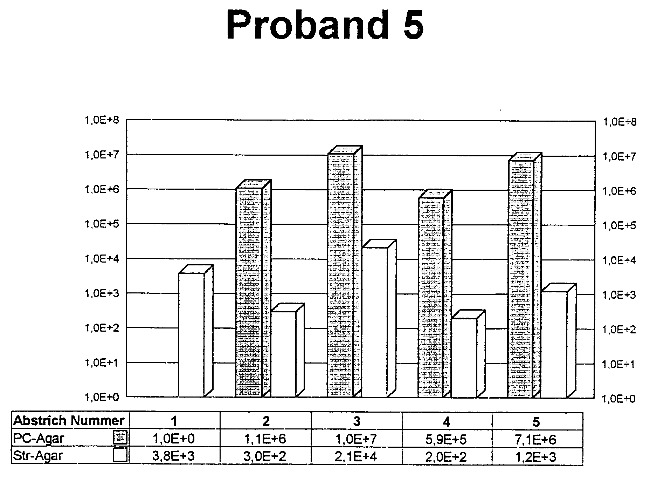

In den Figuren 1 bis 5 sind die Ergebnisse für verschiedene Probanden dargestellt, die einer Testreihe zur Untersuchung des selektiven Einflusses von SMK auf die Anzahl der β-hämolysierender Streptococcen in der Mundflora unterzogen wurden.

### Beispiele:

Die erfindungsgemäße Verwendung von SMK zur Herstellung einer Zusammensetzung und zur Behandlung von durch β-hämolysierende Streptococcen hervorgerufene Erkrankungen wird nachfolgend unter Bezugnahme auf Beispiele erläutert.

Fünf Probanden wurden einer Testreihe zur Untersuchung des Einflusses von SMK auf die Anzahl der β-hämolysierender Streptococcen in der Mundflora unterzogen. Alle Probanden waren männlichen Geschlechts. Weitere Daten zu den Probanden sind in Tabelle 1 angegeben:

**Tabelle 1**

| Proband Nummer | Alter (Jahre) | Größe (cm) | Gewicht (kg) | Raucher / Nichtraucher |
|---|---|---|---|---|
| 1 | 24 | 174 | 61 | NR |
| 2 | 26 | 179 | 70 | R |
| 3 | 29 | 175 | 62 | NR |
| 4 | 45 | 176 | 62 | R |
| 5 | 25 | 187 | 82 | NR |

Die Verabreichung von SMK erfolgte in Form von Floracit®-Gummetten (IMPULS Arzneimittelvertrieb GmbH, D-21465 Reinbek). Diese kommerziell erhältlichen SMK - Lutschtabletten enthalten je Lutschtablette zu 750 mg als wirksame Bestandteile Süßmolkepulver 359,2 mg, Lactose 151,2 mg, Tricalciumphosphat 12,6 mg.

Die Testreihen wurden über einen Zeitraum von 12 Tagen durchgeführt. Am ersten Tag wurden aus dem Mundraum jedes Probanden Abstriche (Nullproben) entnommen. Der Beginn der Einnahme von Floracit-Gummetten erfolgte am 3. Tag. Es wurden täglich über den Tag verteilt 10 Gummetten verabreicht.

Weitere Abstriche während der Behandlung erfolgte am 4., am 6. und am 8. Tag der Untersuchungsreihe. Ab dem 9. Tag wurden keine Gummetten mehr verabreicht. Die Schlußprobe (5. Abstrich) wurde am 12. Tag der Untersuchung entnommen.

Die Abstriche wurden mit sterilen Wattetupfern (reine Baumwolle) entnommen. Nach den Abstrichen wurde der Wattetupfer in 10 ml sterile Ringer-Lösung (Merck Artikel Nr. 15525) gebracht, gut durchgeschüttelt mit einem Ultraschallbad (Transsonic T310, Frequenz 35 kHz) behandelt und einer dekadischen Reihenverdünnung unterworfen.

Von den Verdünnungsstufen wurden vorherbestimmte Mengen auf vorbereitete Petrischalen mit unterschiedlichen Nährböden pipettiert und verteilt, wie nachfolgend detaillierter erläutert wird. Dann wurden die Petrischalen bei 37°C 48 bis 72 Stunden bebrütet und anschließend ausgezählt.

Zur Bestimmung der Gesamtkeimzahl wurden Petrischalen verwendet, die 15 ml Plate-Count-Agar (PC-Agar, Merck-Artikel 5464) als Nährmedium enthielten. PC-Agar entspricht den Vorschriften von § 35 LMBG und enthält 5g/l Pepton aus Gasein, 2,5 g/l Hefeextrakt, 1 g/l Glucose und 14 g/l Agar-Agar.

Zur selektiven Isolierung β-hämolysierender Streptococcen aus stark mit Begleitkeimen durchsetzten Abstrichen wurde β-Streptococcen-Elektiv-Agar nach Liebermeister und Braveny (Str-Agar, Merck-Artikel 10406) verwendet. Dieser Nährboden enthält 1 g/l Pepton aus Fleisch, 0,6 g/l Fleischextrakt, 5 g/l Hefeextrakt, 0,02 g/l Lysin, 6 g/l Natriumchlorid, 2 g/l Natriumhydrogenphosphat und 15 g/l Agar-Agar.

Die Ergebnisse sind unten in Tabelle 2 dargestellt. Die mit PC-Agar gekennzeichneten Daten geben die gemessene Gesamtkeimzahl der Probanden an, während die mit Str-Agar gekennzeichneten Werte die bestimmte Keimzahl der β-hämolysierende Streptococcen angibt.

**Tabelle 2**

| | Proband 1 | | Proband 2 | | Proband 3 | | Proband 4 | | Proband 5 | |
|---|---|---|---|---|---|---|---|---|---|---|
| Tag | PC | STR | PC | STR | PC | STR | PC | STR | PC | STR |
| 1 | 1,0 | 58,0 | 0,2 | 15,0 | 0,3 | 5,4 | 0,1 | 2,7 | - | 3,8 |
| 4 | 7,8 | 39,0 | 1,4 | 0,6 | - | - | 3,0 | 0,3 | 1,1 | 0,3 |
| 6 | 36,0 | 3,3 | 1,3 | 34,5 | 2,1 | 5,8 | 8,4 | 0,8 | 10,7 | 21,3 |
| 8 | 10,9 | 0,1 | 3,4 | 1,5 | 0,2 | 0,7 | 2,3 | 0,1 | 0,6 | 0,2 |
| 12 | 6,2 | 8,3 | 1,0 | 7,8 | 4,1 | 1,0 | 7,2 | 7,2 | 7,1 | 1,2 |

Anmerkung:
Werte für PC-Agar *10⁶
Werte für STR-Agar *10³

Ferner sind die Ergebnisse für jeden Probanden als Grafik in den Figuren 1 bis 5 dargestellt. Die dunkeln Säulen kennzeichnen die Keimzahl auf PC-Agar und die hellen Säulen geben die Keimzahl auf Str-Agar an. Zur Verbesserung der Darstellbarkeit von Werten über mehrere Größenordnungen in einer Graphik wurde für die Keimzahl eine logarithmische Achseneinteilung gewählt.

### Bewertung der Ergebnisse:

Proband 1: Die Gesamtkeimzahl (PC-Agar) lag in allen Abstrichen zwischen 10⁶ und 3,6*10⁷, die Streptococcen-Zahl war zu Beginn des Test hoch, sank während der Anwendung des SMK deutlich ab und erreicht nach dem Absetzen wieder nahezu den Anfangswert.
Proband 2: Die Gesamtkeimzahl war auch hier relativ konstant, während die Streptococcen während des Tests deutlich (Abstriche 2 und 4) bzw. leicht (Abstrich 3) sanken. Hier wird der Anfangswert nach dem Absetzen der Floracit-Gummetten nicht wieder erreicht.
Proband 3: Der Proband war zum Abstrich 2 verhindert (für die Graphik wurde ein fiktiver Wert von jeweils 1*10⁰ eingegeben). Die Gesamtkeimzahl schwankt hier zwischen 2,2*10⁵ und 4*10⁶, die Streptococcenzahl sinkt von 5,4*10³ auf 7*10² und stagniert nach dem Absetzen.
Proband 4: Während der Anfangswert für die Gesamtkeimzahl relativ niedrig ist, stellt sich während des Tests eine konstante Gesamtkeimzahl ein. Die Streptococcenzahl sinkt deutlich und erreicht nach dem Absetzen einen höheren Wert als zu Beginn des Tests.
Proband 5: Bei Proband 5 war der Wert der Gesamtkeimzahl für den Startwert so hoch, daß er nicht auswertbar war (für die Graphik wurde 1*10⁰ angegeben, s.o.). Die Gesamtkeimzahlen schwankten stark, die Streptococcenzahl wurde beim 2. und 4. Abstrich deutlich reduziert und stieg nach dem Absetzen wieder an. Der 3. Abstrich fällt aus dem Rahmen, nach Aussage des Probanden hatte er kurz vor dem Abstrich "ein reichhaltiges Essen" zu sich genommen.

Wie aus den obigen Beispielen ersichtlich ist, besitzt SMK einen selektiven Effekt auf die Keimzahl unerwünschter β-hämolysierender Streptococcen, die während der Behandlung deutlich vermindert werden konnten. Nach dem absetzen der Süßmolke wurde ein Wiederanstieg der Keimzahlen auf dem Streptococcen-Agar beobachtet. Einen Einfluß der Süßmolke auf die Gesamtkeimzahl war hingegen kaum festzustellen, die erhaltenen Schwankungen sind hier eher auf die individuellen Lebensumstände der Probanden zurückzuführen.

## Patentansprüche

1. Verwendung von Süßmolkekonzentrat zur Herstellung einer Zusammensetzung zur selektiven Bekämpfung β-hämolysierender Streptococcen und der durch diese verursachten Erkrankungen.

2. Verwendung gemäß Anspruch 1, wobei die β-hämolysierenden Streptococcen vom Typ Streptococcus mutans sind.

3. Verwendung der Zusammensetzung gemäß Anspruch 1 oder 2, wobei die Zusammensetzung zur Behandlung von Erkrankungen bei Menschen verwendet wird.

4. Verwendung der Zusammensetzung gemäß mindestens einer der Ansprüche 1 bis 3, wobei die Erkrankungen Karieserkrankungen sind.

5. Verwendung gemäß mindestens einem der Ansprüche 1 bis 4, wobei das Süßmolkekonzentrat ein Konzentrat aus teilweise oder vollständig hydrolysierter Süßmolke ist.

6. Verwendung gemäß mindestens einem der Ansprüche 1 bis 5, wobei das Süßmolkekonzentrat aus Kuhmilch, Ziegenmilch, Schafsmilch, Büffelmilch oder Kamelmilch gewonnen wird.

7. Verwendung gemäß mindestens einem der Ansprüche 1 bis 6, wobei die Zusammensetzung ferner Zusatzstoffe enthält, die zur Herstellung von Lebensmitteln, insbesondere diätetischen Lebensmitteln, und Nahrungsergänzungsmitteln geeignet sind.

8. Verwendung gemäß mindestens einem der Ansprüche 1 bis 7, wobei die Zusammensetzung ferner pharmazeutisch annehmbare Zusatzstoffe und/oder Trägermaterialien umfaßt.

9. Verwendung gemäß mindesten einem der Ansprüche 1 bis 8, wobei die Zusammensetzung in fester Form als Lutschtablette, Pulver oder Granulat, oder in flüssiger Form als Sirup oder Saft vorliegt.
